(19) **Europäisches Patentamt**
**European Patent Office**
**Office européen des brevets**

(11) **EP 3 309 792 A1**

(12) **EUROPÄISCHE PATENTANMELDUNG**

(43) Veröffentlichungstag:
**18.04.2018 Patentblatt 2018/16**

(51) Int Cl.:
***G16H 30/20*** *(2018.01)* ***G16H 40/63*** *(2018.01)*

(21) Anmeldenummer: **17190742.1**

(22) Anmeldetag: **13.09.2017**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD**

(30) Priorität: **14.10.2016 DE 102016220093**

(71) Anmelder: **Siemens Healthcare GmbH**
**91052 Erlangen (DE)**

(72) Erfinder:
• **Flohr, Thomas**
**91486 Uehlfeld (DE)**
• **Schmidt, Bernhard**
**90766 Fürth (DE)**

(54) **BESTIMMEN EINES AUFNAHMEPARAMETERS FÜR EIN BILDGEBENDES VERFAHREN**

(57) Die Erfindung basiert darauf, dass ein patientenspezifischer Aufnahmewert für eine Aufnahme eines Bilddatensatzes empfangen wird. Durch Anwendung einer trainierten Abbildungsvorschrift auf den patientenspezifischen Aufnahmewert wird ein Aufnahmeparameter bestimmt. Hierbei basiert die trainierte Abbildungsvorschrift auf einer Mehrzahl von Trainings-Datensätzen, wobei die Trainings-Datensätze jeweils wenigstens einen patientenspezifische Trainings-Aufnahmewert und wenigstens einen Trainings-Aufnahmeparameter sowie wenigstens eine Trainings-Qualitätsbewertung umfassen. Die Erfinder haben erkannt, dass es hiermit, die komplexen Einflüsse der patientenspezifischen Aufnahmewerte und der Aufnahmeparameter auf das Ergebnis der Bildgebung zu quantifizieren. Durch die Vergabe der Trainings-Qualitätsbewertung durch einzelne Bedienpersonen oder spezifische Gruppen von Bedienpersonen ist es möglich, die Bildaufnahmeparameter flexibel und individuell anzupassen. Insbesondere kann der Aufnahmeparameter derart bestimmt werden, dass das Ergebnis der Bildgebung individuell an eine Bedienperson angepasst ist.

FIG 4

EP 3 309 792 A1

**Beschreibung**

Bei verschiedenen medizinischen Bildaufnahmegeräten müssen zur Erzielung optimaler Untersuchungsergebnisse zahlreiche technische Parameter (sog. Bildaufnahmeparameter) festgelegt oder eingestellt werden. Diese Bildaufnahmeparameter steuern die Datenaufnahme und/oder die Bildrekonstruktion und beeinflussen so den erzeugten Bilddatensatz. Die Bildaufnahmeparameter können hierbei durch die Wahl vorgegebener Standardeinstellungen oder Protokolle, die für die gewählte Untersuchung geeignet sind, festgelegt werden. Weiterhin können die einzelnen technischen Parameter auch von der Bedienperson eingestellt oder verändert werden.

In der Computertomographie (kurz CT) betreffen diese Bildaufnahmeparameter unter anderem Spannung und Strom der verwendeten Röntgenröhre, Einstellungen für die automatische Dosismodulation, Geschwindigkeit des Tischvorschub, Schichtdicke der untersuchten Schichten oder Parameter für die Bildrekonstruktion. In der Magnetresonanztomographie (kurz MR) betreffen diese Bildaufnahmeparameter unter anderem die verwendete Untersuchungssequenz, die Stärke des Magnetfeldes oder die Frequenz der überlagerten Radiowellen.

Es ist bekannt, Standardeinstellungen für Bildaufnahmeparameter abhängig von der gewählten Untersuchungsart werkseitig als Protokolle vorzugegeben und im medizinischen Bildaufnahmegerät voreingestellt auszuliefern. Vorgegebene Untersuchungsarten in der CT umfassen beispielsweise die Aufnahme eines Patientenkopfes, die Aufnahme einer Patientenlunge, die Angiographie oder das Drei-Phasen-CT für die Leber. Vorgegebene Untersuchungsarten in der MR umfassen beispielsweise Echtzeit-MR, MR-Spektroskopie oder diffusionsgewichtete MR. Entspricht der mit den vorgegebenen Standardeinstellungen erhaltene Bilddatensatz nicht den spezifischen Anforderungen der Bedienperson an die Qualität, so muss er die Einstellung der Bildaufnahmeparameter selbst oder mit Hilfe eines Spezialisten ändern.

Der Auswirkungen einer Änderung der Bildaufnahmeparameter auf die erreichte Qualität des Bilddatensatzes ist aber sehr komplex und für die Bedienperson nicht oder nur mit erheblichem Aufwand erfassbar oder nachvollziehbar. Die Bedienperson kann daher die Bildaufnahmeparameter nicht effizient und zielgerichtet ändern, um die Qualität des Bilddatensatzes zu erhöhen. Werden trotzdem Bildaufnahmeparameter manuell geändert, führen die dadurch verursachten Änderungen oft nicht zu einer Verbesserung der Qualität des Bilddatensatzes. Eine werkseitige Anpassung der Parameter ist nur eingeschränkt möglich, da es unterschiedliche Anforderungen an die Qualität des Bilddatensatzes für unterschiedliche medizinische Fragestellungen und Bedienpersonen gibt. Erschwerend ist weiterhin, dass für eine Bedienperson oftmals nur feststellbar ist, dass die Qualität des erzeugten Bilddatensatzes nicht den Erwartungen entspricht, nicht aber der Grund (z.B. mangelnde Auflösung, Bildartefakte) für die mangelnde Qualität angegeben werden kann.

Es ist daher die Aufgabe der vorliegenden Erfindung, ein Verfahren bereitzustellen, welches eine flexible und individuelle Anpassung der Bildaufnahmeparameter ermöglicht, insbesondere für die Bedienperson.

Diese Aufgabe wird gelöst durch ein Verfahren nach Anspruch 1, durch eine Parameterbestimmungseinheit nach Anspruch 10 sowie durch ein Computerprogrammprodukt nach Anspruch 12, durch ein computerlesbares Medium nach Anspruch 13 sowie durch ein bildgebendes Gerät nach Anspruch 14.

Nachstehend wird die erfindungsgemäße Lösung der Aufgabe in Bezug auf die beanspruchten Vorrichtungen als auch in Bezug auf das beanspruchte Verfahren beschrieben. Hierbei erwähnte Merkmale, Vorteile oder alternative Ausführungsformen sind ebenso auch auf die anderen beanspruchten Gegenstände zu übertragen und umgekehrt. Mit anderen Worten können die gegenständlichen Ansprüche (die beispielsweise auf eine Vorrichtung gerichtet sind) auch mit den Merkmalen, die in Zusammenhang mit einem Verfahren beschrieben oder beansprucht sind, weitergebildet sein. Die entsprechenden funktionalen Merkmale des Verfahrens werden dabei durch entsprechende gegenständliche Module ausgebildet.

Weiterhin sind im Folgenden Merkmale, Vorteile oder alternative Ausführungsformen, die im Zusammenhang mit den Trainingsgrößen beschrieben sind, auf die Anwendungsgrößen zu übertragen und umgekehrt. Insbesondere sind Merkmale, Vorteile oder alternative Ausführungsformen des patientenspezifischen Trainings-Aufnahmewertes auf den patientenspezifischen Aufnahmewert zu übertragen und umgekehrt. Insbesondere sind Merkmale, Vorteile oder alternative Ausführungsformen des Trainings-Aufnahmeparameters auf den Aufnahmeparameter zu übertragen und umgekehrt. Insbesondere sind Merkmale, Vorteile oder alternative Ausführungsformen der Trainings-Qualitätsbewertung auf die Qualitätsbewertung zu übertragen und umgekehrt. Insbesondere sind Merkmale, Vorteile oder alternative Ausführungsformen eines Trainings-Bilddatensatzes auf den Bilddatensatz zu übertragen und umgekehrt. Insbesondere sind Merkmale, Vorteile oder alternative Ausführungsformen eines Trainings-Datensatzes auf den Ergebnis-Datensatz zu übertragen und umgekehrt.

Das erfindungsgemäße Verfahren zum Bestimmen eines Aufnahmeparameters umfasst das Empfangen wenigstens eines patientenspezifischen Aufnahmewertes für eine Aufnahme eines Bilddatensatzes. Weiterhin umfasst das erfindungsgemäße Verfahren das Bestimmen eines Aufnahmeparameters durch Anwendung einer trainierten Abbildungsvorschrift auf den patientenspezifischen Aufnahmewert, wobei die trainierte Abbildungsvorschrift auf einer Mehrzahl von Trainings-Datensätzen basiert, und wobei die Trainings-Datensätze jeweils wenigstens einen patientenspezifische Trainings-Aufnahmewert und wenigstens einen Trainings-Aufnahmeparameter sowie wenigstens eine Trainings-Qualitätsbewertung umfassen. Das Verfahren kann insbesondere ein computerimplementiertes Verfahren sein. Die Erfinder haben erkannt, dass es durch das Anwenden einer trainierten Abbildungsvorschrift anhand von Trainings-Datensätzen möglich ist, die komplexen Einflüsse der patientenspezifischen Aufnahmewerte und der Aufnahmeparameter auf das Ergebnis der Bildgebung zu quantifizieren. Wird die Trainings-Qualitätsbewertung durch nur eine Bedienperson durchgeführt, ist es dadurch möglich, die Bildaufnahmeparameter flexibel und individuell anzupassen. Insbesondere kann der Aufnahmeparameter aus dem patientenspezifischen Aufnahmewerten derart bestimmt werden, dass das Ergebnis der Bildgebung individuell an eine Bedienperson angepasst ist.

Gemäß einem weiteren Aspekt der Erfindung wird weiterhin ein Bilddatensatz unter Verwendung des Aufnahmeparameters mittels eines bildgebenden Geräts aufgenommen. Für diesen Bilddatensatz wird ein Ergebnis-Datensatz bestimmt, der eine Qualitätsbewertung des Bilddatensatzes und den patientenspezifischen Aufnahmewert und den Aufnahmeparameter umfasst. Dieser Ergebnis-Datensatz wird gespeichert. Die Erfinder haben erkannt, dass durch das Speichern des Ergebnis-Datensatzes ein zusätzlicher, dem Training zu Grunde liegende Datensätze erzeugt werden können. Dies führt zu einer präziseren Bestimmung der Abbildungsvorschrift.

Gemäß einem weiteren Aspekt der Erfindung wird weiterhin die trainierte Abbildungsvorschrift basierend auf den Trainings-Datensätzen sowie dem Ergebnis-Datensatz trainiert. Insbesondere erfolgt das Trainieren der trainierten Abbildungsvorschrift als Reaktion auf das Speichern des Ergebnis-Datensatzes. Die Erfinder haben erkannt, dass ein erneutes Training der Abbildungsvorschrift nach jeder Untersuchung dazu geeignet ist, zeitliche Änderungen der spezifischen Anforderungen der Bedienperson an die Qualität zu erfassen. Damit kann die trainierte Abbildungsvorschrift daran angepasst werden.

Gemäß einem weiteren Aspekt der Erfindung beurteilt die Qualitätsbewertung wenigstens die Qualität des Bilddatensatzes, die Zeitdauer der Aufnahme des Bilddatensatzes und/oder die Strahlendosis entsprechend der Aufnahme des Bilddatensatzes vom Patienten. Gemäß einem weiteren Aspekt der Erfindung ist die Trainings-Qualitätsbewertung ein Maß für die Qualität eines Trainings-Bilddatensatzes, die Zeitdauer der Aufnahme des Trainings-Bilddatensatzes und/oder die Strahlendosis entsprechend der Aufnahme des Trainings-Bilddatensatzes von einem Patienten. Die Erfinder haben erkannt, dass diese drei Einflussfaktoren maßgeblich den Erfolg einer Untersuchung quantifizieren und daher als Qualitätsbewertung für das Training eines selbstlernenden Systems besonders gut geeignet sind. Eine gute Bildqualität führt dazu, dass keine weiteren bildgebenden Untersuchungen notwendig sind, eine kurze Dauer der Aufnahme führt zu einer besseren Auslastung des bildgebenden Gerätes. Eine geringe durch die Aufnahme des Bilddatensatzes aufgenommene Strahlendosis führt zu einer geringeren Schädigung des Patientengewebes durch die Aufnahme. Eine durch eine Bedienperson bestimmte Qualitätsbewertung oder Trainings-Qualitätsbewertung heißt positiv, falls die Bedienperson überwiegend zufrieden mit der Aufnahme des Bilddatensatzes ist. Eine durch eine Bedienperson bestimmte Qualitätsbewertung oder Trainings-Qualitätsbewertung heißt negativ, falls die Bedienperson überwiegend unzufrieden mit der Aufnahme des Bilddatensatzes ist.

Gemäß einem weiteren Aspekt der Erfindung umfassen die Qualitätsbewertung und/oder die Trainings-Qualitätsbewertung einen diskreten Parameter, der nur endlich viele Werte annehmen kann. Die Erfinder haben erkannt, dass eine Bewertung einer Aufnahme eines Bilddatensatzes mittels einer diskreten Skala den technischen Fähigkeiten einer Bedienperson entspricht. Bei der Bewertung mit einer kontinuierlichen Skala entsprechen mehrere Werte der kontinuierlichen Skala der gleichen Qualitätsbewertung der Bedienperson. Dies kann negative Auswirkungen auf das Training der trainierten Abbildungsvorschrift haben. Bei einer diskreten Skala kann es sich beispielsweise um Noten handeln, oder eine Bewertung mittels Worten (wie z.B. "ungeeignet", "wenig geeignet", "etwas geeignet", "geeignet"). Bei einer diskreten Skala kann es sich auch um eine Farbskala handeln, insbesondere um zwei oder drei Farben einer Ampel, beispielsweise "rot" und "grün" oder "rot", "gelb" und "grün".

Gemäß einem weiteren Aspekt dieser Erfindung kann der diskrete Parameter genau zwei verschiedene Werte annehmen. In anderen Worten ist der diskrete Parameter eine binäre Größe. Diese binäre Größe erfasst die Gesamtbewertung der Aufnahme des Bilddatensatzes und nimmt Werte an, die "unzufrieden mit der Aufnahme des Bilddatensatzes" oder "zufrieden mit der Aufnahme des Bilddatensatzes" repräsentieren. Die Erfinder haben erkannt, dass eine Bedienperson oftmals zwar feststellt, dass die Aufnahme des Bilddatensatzes nicht den Anforderungen entspricht. Die Bedienperson kann aber nicht feststellen, warum die Aufnahme des Bilddatensatzes nicht den Anforderungen entspricht. Mittels des vorgeschlagenen binären Parameters ist es für die Bedienperson in dieser Situation aber trotzdem möglich, die bildgebende Untersuchung zu bewerten.

Gemäß einem weiteren Aspekt der Erfindung wird die trainierte Abbildungsvorschrift mittels einer Stützvektormaschine bestimmt, wobei in der Trainingsphase Datensätze gemäß dem diskreten Parameter in wenigstens eine erste und eine zweite Kategorie sortiert werden, und wobei im Parameterraum aller Kombinationen patientenspezifischer Aufnahmewerte und Aufnahmeparameter wenigstens eine erste Hyperfläche bestimmt wird, welche die erste und die zweite Kategorie trennt. Weiterhin wird die trainierte Abbildungsvorschrift wenigstens mittels der ersten Hyperebene bestimmt. Die Erfinder haben erkannt, dass durch die Hyperfläche der Parameterraum in zwei oder mehr Unterräume eingeteilt wird. Jedem dieser Unterräume kann die voraussichtliche Qualitätsbewertung zugeordnet werden, die eine Bedienperson für die Kombination aus patientenspezifischen Aufnahmewert und Aufnahmeparameter aus diesem Unterraum zuweist. Auf Grundlage dieser Unterräume kann daher ein Aufnahmeparameter aus dem patientenspezifischen Aufnahmewert bestimmt werden, der die Bedienperson mit hoher Wahrscheinlichkeit zufrieden stellt.

Gemäß einem weiteren Aspekt der Erfindung wird die Abbildungsvorschrift derart durch die Hyperfläche bestimmt, dass sie einen patientenspezifischen Aufnahmewert derart auf den Aufnahmeparameter abbildet, dass der Abstand zwischen der ersten Hyperfläche und einem Datenpunkt extremal wird, wobei der Datenpunkt aus dem patientenspezifischen Aufnahmewert und dem bestimmten Aufnahmeparameter besteht. Hierbei werden nur Aufnahmeparameter berücksichtigt, die zur Aufnahme des Bilddatensatzes in den Grenzen der Gerätespezifikationen eingestellt werden können. Hierbei heißt extremal maximal, falls der Datenpunkt in einem Unterraum liegt, der einer Qualitätsbewertung entspricht, die eine Bedienperson für eine ihn zufrieden stellende Aufnahme vergibt. Dagegen bedeutet extremal minimal, falls der Datenpunkt in einem Unterraum liegt, der einer Qualitätsbewertung entspricht, die eine Bedienperson für eine ihn nicht zufrieden stellende Aufnahme vergibt. Die Erfinder haben erkannt, dass durch diese Wahl des Aufnahmeparameters die Wahrscheinlichkeit am größten ist, dass das Ergebnis der Aufnahme den Erwartungen der Bedienperson entspricht, da der Datenpunkten so möglichst weit in beziehungsweise möglichst nah bei den Datenpunkten liegt, die eine positive Bewertung durch die Untersuchungsperson versprechen lassen.

Weiterhin betrifft die Erfindung eine Parameterbestimmungseinheit zur Bestimmung eines Aufnahmeparameters, umfassend folgende Einheiten:

- Schnittstelle, ausgebildet zum Empfangen eines patientenspezifischen Aufnahmewertes,
- Recheneinheit, ausgebildet zum Bestimmen eines Aufnahmeparameters durch Anwendung einer trainierten Abbildungsvorschrift auf den patientenspezifischen Aufnahmewert, wobei die trainierte Abbildungsvorschrift auf einer Mehrzahl von Trainings-Datensätzen basiert, wobei die Trainings-Datensätze jeweils wenigstens einen patientenspezifische Trainings-Aufnahmewert und wenigstens einen Trainings-Aufnahmeparameter sowie wenigstens eine Trainings-Qualitätsbewertung umfassen.

Eine solche Parameterbestimmungseinheit kann insbesondere dazu ausgebildet sein das zuvor beschriebenen erfindungsgemäßen Verfahren und seine Aspekte auszuführen. Die Parameterbestimmungseinheit ist dazu ausgebildet dieses Verfahren und seine Aspekte auszuführen, indem die Schnittstelle und die Recheneinheit ausgebildet sind, die entsprechenden Verfahrensschritte auszuführen. Die Erfindung betrifft weiterhin ein bildgebendes Gerät umfassend eine erfindungsgemäße Parameterbestimmungseinheit.

Die Erfindung betrifft auch ein Computerprogrammprodukt mit einem Computerprogramm sowie ein computerlesbares Medium. Eine weitgehend softwaremäßige Realisierung hat den Vorteil, dass auch schon bisher verwendete Steuereinrichtungen auf einfache Weise durch ein Software-Update nachgerüstet werden können, um auf die erfindungsgemäße Weise zu arbeiten. Ein solches Computerprogrammprodukt kann neben dem Computerprogramm gegebenenfalls zusätzliche Bestandteile wie z. B. eine Dokumentation und/oder zusätzliche Komponenten auch Hardware-Komponenten, wie z.B. Hardware-Schlüssel (Dongles etc.) zur Nutzung der Software, umfassen.

Ein patientenspezifischer Aufnahmewert ist ein Parameter, der für die Bildgebung eines Patienten vorgegeben ist und durch die Bedienperson des bildgebenden Gerätes nicht festgelegt werden kann. Mit anderen Worten kann der patientenspezifische Aufnahmewert von der Bedienperson nicht beeinflusst werden, insbesondere ist der patientenspezifische Aufnahmewert bereits vor dem Beginn der Bildgebung bzw. der Aufnahme festgelegt. Ein patientenspezifischer Aufnahmewert kann insbesondere die Bauart des Bildaufnahmegeräts, die gewählte Untersuchungsart, eine Verdachtsdiagnose, das untersuchte Organ, das Geschlecht des untersuchten Patienten, das Gewicht des untersuchten Patienten und/oder das Alter des untersuchten Patienten betreffen. Betrifft der patientenspezifische Aufnahmewert mehrere Größen, kann er insbesondere als Vektor realisiert sein. Insbesondere ist der patientenspezifische Aufnahmewert kein den Patienten betreffender Bilddatensatz.

Ein Aufnahmeparameter ist ein Parameter, der für die Bildgebung eines Patienten mittels eines bildgebenden Gerätes am bildgebenden Gerät eingestellt werden kann. Ein Aufnahmeparameter kann insbesondere einen Datenaufnahmeparameter und/oder einen Bildrekonstruktionsparameter betreffen. Ein Datenaufnahmeparameter kann insbesondere die Röhrenspannung bei einem CT-Gerät, den Röhrenstrom bei einem CT-Gerät, die automatische Dosismodulation, den Tischvorschub und/oder die Schichtdicke betreffen. Ein Bildrekonstruktionsparameter kann insbesondere den Faltungskern des Bildrekonstruktionsalgorithmus oder die Fenstereinstellung des Bildrekonstruktionsalgorithmus betreffen. Betrifft der Aufnahmeparameter mehrere Größen, kann er insbesondere als Vektor realisiert sein.

Ein Bilddatensatz bezeichnet ein oder mehrere Bilder eines Patienten, die durch eine bildgebende Untersuchung bestimmt wurden. Insbesondere kann es sich um zweidimensionale oder dreidimensionale Bilder handeln. Ein aus einem patientenspezifischen Aufnahmewert und einem Aufnahmeparameter resultierender Bilddatensatz bezeichnet einen Bilddatensatz, der unter Verwendung dieses patientenspezifischen Aufnahmewertes und dieses Aufnahmeparameters bestimmt wurde.

Eine Qualitätsbewertung eines Bilddatensatzes ist ein Parameter für die Qualität eines Ergebnisses einer Bildgebung, die unter Verwendung eines patientenspezifischen Aufnahmewertes und eines Aufnahmeparameters durchgeführt wurde. Eine Trainings-Qualitätsbewertung eines Trainings-Bilddatensatzes ist ein Parameter für die Qualität des Ergebnisses einer Trainings-Bildgebung, die unter Verwendung eines patientenspezifischen Trainings-Aufnahmewertes und eines Trainings-Aufnahmeparameters durchgeführt wurde. Die Qualität eines Bilddatensatzes kann insbesondere ein Maß für die Auflösung und/oder für den Kontrast des Bilddatensatzes sein. Eine Qualitätsbewertung kann aber insbesondere auch andere Parameter der Untersuchung betreffen, beispielsweise deren Dauer oder verwendete Untersuchungsdosis. In anderen Worten kann eine Qualitätsbewertung eines Bilddatensatzes nicht nur den Bilddatensatz an sich bewerten, sondern auch die Umstände der Aufnahme des Bilddatensatzes. Bei einem Qualitätsparameter kann es sich um eine Zahl handeln, aber auch um einen binären Wert. Insbesondere kann der binäre Wert abbilden, ob eine Bedienperson mit der Qualität des Bilddatensatzes zufrieden ist. Weiterhin kann es sich bei dem Qualitätsparameter um einen Vektor handeln, bei dem jeder Eintrag einen anderen Aspekt der Qualität eines Bilddatensatzes bewertet.

Eine trainierte Abbildungsvorschrift ist eine Funktion, die einen patientenspezifischen Aufnahmewert auf einen Aufnahmeparameter abbildet, und die basierend auf mehreren Trainings-Datensätzen bestimmt wurde. Dabei umfassen die Trainings-Datensätze jeweils einen patientenspezifischen Trainings-Aufnahmewert, einen Trainings-Aufnahmeparameter und eine Trainings-Qualitätsbewertung. Dabei misst die Trainings-Qualitätsbewertung die Qualität eines Bilddatensatzes, der unter Verwendung des patientenspezifischen Trainings-Aufnahmewertes und des Trainings-Aufnahmeparameters bestimmt wurde. Die trainierte Abbildungsvorschrift soll einen patientenspezifischen Aufnahmewert derart auf einen Aufnahmeparameter abbilden, dass ein mit patientenspezifischen Aufnahmewert und dem bestimmten Aufnahmeparameter aufgenommene Bilddatensatz mit hoher Wahrscheinlichkeit den Anforderungen der Bedienperson (ausgedrückt durch die Qualitätsbewertung) entspricht.

Eine Stützvektormaschine (kurz SVM) ist eine Methode, um in einem Parameterraum von kategorisierten Trainings-Datensätzen eine Hyperebene oder eine Hyperfläche zu bestimmen, welche die einzelnen Kategorien der Trainings-Datensätze trennt. Für den Begriff Stützvektormaschine ist auch der englischsprachige Begriff Support Vector Machine geläufig. Die Hyperfläche wird derart bestimmt, dass der Abstand der am nächsten an der Hyperfläche liegenden Datenpunkte maximiert wird. Im Allgemeinen kann eine Stützvektormaschine nur Hyperebenen bestimmen, zur Bestimmung einer Hyperfläche werden die Trainings-Datensätze in einen höherdimensionalen Parameterraum überführt. In diesem höherdimensionalen Parameterraum kann dann eine trennende Hyperebene bestimmt werden, die bei Rücktransformation zu einer Hyperfläche deformiert. Die Bestimmung der trennenden Hyperebene kann auch im höherdimensionalen Parameterraum durch Anwendung einer positiv-definiten Kernelfunktion sehr effizient durchgeführt werden. Durch Einführung eines Schlupfparameters erhält man Hyperflächen, welche die Kategorien nicht perfekt trennen. Hiermit kann eine Überanpassung an die Trainings-Datensätze vermieden werden. Eine Stützvektormaschine ist dem Fachmann bekannt, daher wird auf weitere Ausführungen verzichtet, es wird hierzu auf die Monographie Ingo Steinwart, Andreas Christmann "Support Vector Machines" Springer (2008) verwiesen.

Ein Parameterraum ist ein $(M + N)$-dimensionaler Vektorraum aller Kombinationen oder Datensätze von patientenspezifischen Aufnahmewerten und von Aufnahmeparametern, wenn der patientenspezifische Aufnahmewert durch einen Vektor mit $M$ Einträgen realisiert wird, und der Aufnahmeparameter durch einen Vektor mit $N$ Einträgen realisiert wird. Betrifft der patientenspezifische Aufnahmewert nur eine Größe, so ist $M$ gleich 1. Betrifft der Aufnahmeparameter nur eine Größe, so ist $N$ gleich 1. Eine bestimmte Kombination oder Datensatz aus einem patientenspezifischen Aufnahmewert und einem Aufnahmeparameter entspricht einem Punkt bzw. einem Vektor im $(M+N)$-dimensionalen Vektorraum aller Kombinationen.

Eine Kategorie von Trainings-Datensätzen entspricht einer Menge von Trainings-Datensätzen, welche die gleiche oder eine ähnliche Trainings-Qualitätsbewertung aufweisen. Betrifft der Trainings-Qualitätsparameter einen diskreten Parameter mit endlich vielen möglichen Werten, so kann es insbesondere genauso viele Kategorien wie diskrete Werte geben, und die Trainings-Datensätze können den Kategorien entsprechend des diskreten Parameters der Trainings-Qualitätsbewertung zugeordnet werden. Betrifft der Trainings-Qualitätsparameter einen binären Parameter, dann kann es insbesondere nur zwei Kategorien geben, zu welchen die Trainings-Datensätze entsprechend der Trainings-Qualitätsparameter zugeordnet werden können.

Ein Unterraum eines Parameterraums, der eine Kategorie von Trainings-Datensätzen betrifft, ist eine Teilmenge des Parameterraums, der überwiegend nur diese Kategorie von Trainings-Datensätzen enthält. Ein Unterraum ist nicht notwendigerweise linear.

Eine Hyperfläche ist ein $(M+N-1)$-dimensionales Objekt im $(M+N)$-dimensionalen Parameterraum aller Kombinationen von patientenspezifischen Aufnahmewerten und Aufnahmeparametern. In einem 2-dimensionalen Parameterraum entspricht eine Hyperfläche insbesondere einer 1-dimensionalen, möglicherweise gekrümmten Linie in diesem Parameterraum, in einem 3-dimensionalen Parameterraum entspricht eine Hyperfläche insbesondere einer 2-dimensionalen, möglicherweise gekrümmten Oberfläche in diesem Parameterraum. Eine Hyperfläche kann insbesondere durch eine implizite Funktion

$$f(x_1, x_2, \cdots, x_M, y_1, y_2, \cdots, y_N) = 0$$

definiert sein, wobei die Variablen x den M Einträgen des Vektors des patientenspezifischen Aufnahmewertes entsprechen, und die Variablen y den N Einträgen des Vektors des Aufnahmeparameters entsprechen. Eine Hyperfläche hat insbesondere die Eigenschaft, dass sie zwei unterschiedliche Seiten besitzt, wobei für die eine Seite f > 0 gilt, und für die andere Seite f < 0 gilt. Eine Hyperfläche kann insbesondere zwei unterschiedliche Unterräume trennen. Eine Hyperebene ist eine Hyperfläche, deren definierende implizite Funktion linear in allen Variablen ist. Eine Hyperebene ist also insbesondere ein affiner Unterraum des Parameterraums.

Im Folgenden wird die Erfindung anhand der in den Figuren dargestellten Ausführungsbeispiele näher beschrieben und erläutert.

Dabei zeigen

Fig. 1 ein Flussdiagramm

Fig. 2 eine Parameterbestimmungseinheit

Fig. 3 ein MR-Gerät mit einer Parameterbestimmungseinheit

Fig. 4 eine Bewertungseinheit

Fig. 5 einen ersten Parametervektorraum mit Qualitätsbewertungen

Fig. 6 einen zweiten Parametervektorraum mit Qualitätsbewertungen

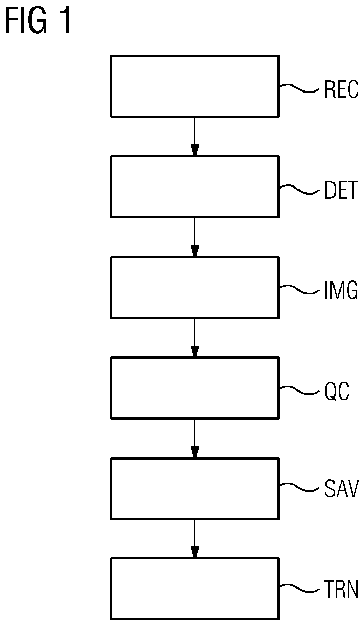

Fig. 1 zeigt ein Flussdiagramm eines Verfahrens zum Bestimmen eines Aufnahmeparameters.

Der erste Schritt des Verfahrens ist das Empfangen REC eines patientenspezifischen Aufnahmeparameters über eine Schnittstelle 201. Im dargestellten Verfahren wird ein patientenspezifischer Aufnahmewert von einer Bedienperson an einem Eingabegerät 204, z.B. einer Tastatur, vorgenommen. Bei einem patientenspezifischen Aufnahmewert handelt es sich z.B. um die Größe, das Gewicht, das Geschlecht oder das Alter des Patienten. Auch kann ein es sich bei einem patientenspezifischen Aufnahmewert um einen Aufnahmebereich handeln, also um einen Teilbereich des Patienten, der aufgenommen werden soll. Alternativ ist es auch möglich, den patientenspezifischen Aufnahmewert aus einer Datenbank abzurufen, in der Daten zum Patienten 303 oder zum aufnehmenden Gerät 300 gespeichert sind. Es können auch mehrere patientenspezifische Aufnahmewerte eingegeben werden oder aus einer Datenbank oder mehreren Datenbanken abgerufen werden.

Der nächste Schritt des Verfahrens ist das Bestimmen DET eines Aufnahmeparameters durch Anwendung einer trainierten Abbildungsvorschrift auf den patientenspezifischen Aufnahmewert, wobei die trainierte Abbildungsvorschrift auf einer Mehrzahl von Trainings-Datensätzen 207.1, 207.2, 207.3 basiert, wobei die Trainings-Datensätze 207.1, 207.2, 207.3 jeweils wenigstens einen patientenspezifische Trainings-Aufnahmewert 208.1, 208.2, 208.3 und wenigstens einen Trainings-Aufnahmeparameter 209.1, 209.2, 209.3 sowie wenigstens eine Trainings-Qualitätsbewertung 210.1, 210.2, 210.3 umfassen. Im dargestellten Verfahren wurde die Abbildungsvorschrift durch Anwendung einer Stützvektormaschine auf die Trainings-Datensätze 207.1, 207.2, 207.3 bestimmt. Eine Trainings-Qualitätsbewertung 210.1, 210.2, 210.3 ist im dargestellten Verfahren ein binärer Parameter, der abbildet, ob die Bedienperson mit der Qualität des Bilddatensatzes 402, der mittels eines patientenspezifischen Trainings-Aufnahmewertes 208.1, 208.2, 208.3 und eines Trainings-Aufnahmeparameters 209.1, 209.2, 209.3 bestimmt wurde, zufrieden oder unzufrieden ist. Anhand der Trainings-Qualitätsbewertung 210.1, 210.2, 210.3 wurden die Trainings-Datensätze 207.1, 207.2, 207.3 in zwei verschiedene Kategorien eingeteilt, wobei die erste Kategorie Trainings-Datensätze 207.1, 207.2, 207.3 mit ausreichender Qualität enthält, und wobei die zweite Kategorie alle Trainings-Datensätze 207.1, 207.2, 207.3 mit nicht ausreichender Qualität Enthält. Die Qualität kann durch eine Bedienperson bewertet werden. Durch Anwendung einer Stützvektormaschine wurde wenigstens eine Hyperfläche 509, 510 bestimmt, welche die beiden Kategorien trennt. Hierbei wurden die Trainings-Datensätze 207.1, 207.2, 207.3 in einen höherdimensionalen Parametervektorraum transformiert, und in diesem eine trennende Hyperebene bestimmt. Die trennende Hyperfläche 509, 510 im ursprünglichen Parametervektorraum ergibt sich dann durch die Rücktransformation der Hyperebene im höherdimensionalen Parametervektorraum in den ursprünglichen Parametervektorraum.

Basierend auf der wenigstens einen Hyperfläche 509, 510 wurde die Abbildungsvorschrift derart bestimmt, dass ein patientenspezifischer Aufnahmewert derart auf einen Aufnahmeparameter abgebildet wird, dass der Abstand des Datenpunkt 512, 602 zu der jeweils nächstliegenden Hyperebene 509, 510 extremal wird, wobei der Datenpunkt aus dem patientenspezifischen Aufnahmewert und dem Aufnahmeparameter besteht. Hierbei wird unterschieden, ob es zu dem patientenspezifischen Aufnahmewert überhaupt einen Aufnahmeparameter gibt, so dass der entstehende Datenpunkt 512 in einem Unterraum mit Datenpunkten mit überwiegend positiven Bewertungen 509 liegt. Ist dies der Fall, so wird der Aufnahmeparameter derart bestimmt, dass der Abstand 513 des Datenpunkts 512 bestehend aus dem patientenspezifischen Aufnahmewert und dem bestimmten Aufnahmeparameter zu der jeweils nächstliegenden Hyperfläche 509 maximal wird. Ist dies nicht der Fall, so wird der Aufnahmeparameter derart bestimmt, dass der Abstand 603 des Datenpunkts 602 bestehend aus dem patientenspezifischen Aufnahmewert und dem bestimmten Aufnahmeparameter zu der jeweils nächstliegenden Hyperfläche 509 minimal wird.

Der Abstand eines Datenpunkts 512, 602 zu einer Hyperfläche 509, 510 wird in diesem Ausführungsbeispiel durch den kleinsten Radius einer Hyperkugel um den Datenpunkt bestimmt, welche die Hyperfläche 509, 510 berührt, aber nicht schneidet. Es sind noch weitere Methoden zur Abstandsbestimmung bekannt, beispielsweise kann die Hyperfläche 509, 510 trianguliert werden, der Abstand vom Datenpunkt 512, 602 zur Hyperfläche ist dann der kleinste Abstand zu einem Segment der triangulierten Hyperfläche. Der Datenpunkt 512, 602 mit maximalem oder minimalem Abstand 513, 603 zu einer Hyperfläche 509, 510 wird in diesem Ausführungsbeispiel mittels einer simulierten Abkühlung berechnet. Für diese Methode ist auch das englische Wort Simulated Annealing geläufig. Es sind aber auch andere Optimierungsmethoden wie der Sintflut- oder der Ameisenalgorithmus bekannt und einsetzbar.

In der gezeigten Ausführungsform basiert die trainierte Abbildungsvorschrift nur auf Trainings-Datensätzen 207.1, 207.2, 207.3, die Trainings-Qualitätsbewertungen 210.1, 210.2, 210.3 einer Bedienperson umfassen. Es ist aber auch möglich, dass die Abbildungsvorschrift auf Trainings-Datensätzen 207.1, 207.2, 207.3 basiert, die Trainings-Qualitätsbewertungen 210.1, 210.2, 210.3 einer bestimmten Gruppe von Bedienpersonen umfassen. Die Gruppe von Bedienpersonen kann ein bestimmtes Merkmal gemeinsam haben, z.B. das Alter der Bedienpersonen oder die Fachrichtung von Ärzten als Bedienpersonen. Die Trainings-Datensätze 207.1, 207.2, 207.3 sind dabei in einer Datenbank 206 gespeichert. In der gezeigten Ausführungsform ist die Datenbank 206 zentral an einem anderen geographischen Ort lokalisiert und über das Internet 205 mit der Parameterbestimmungseinheit 200 verbunden. Die Datenbank 206 kann aber auch in anderer Form mit der Parameterbestimmungseinheit 200 verbunden sein. Weiterhin kann die Parameterbestimmungseinheit 200 die Datenbank 206 auch umfasse, insbesondere kann die Datenbank 206 auch in der Speichereinheit 203 der Parameterbestimmungseinheit 200 gespeichert sein.

Die trainierte Abbildungsvorschrift wird in der gezeigten Ausführungsform auf einem geographisch entfernten Server bestimmt. Dabei wird die trainierte Abbildungsvorschrift vor dem Bestimmen des Aufnahmeparameters an die Parameterbestimmungseinheit 200 übertragen. Die trainierte Abbildungsvorschrift kann aber auch mittels der Parameterbestimmungseinheit 200 bestimmt werden.

Neben der in diesem Ausführungsbeispiel dargestellten Stützvektormaschine können auch andere selbstlernende Systeme verwendet werden, um die trainierte Abbildungsvorschrift zu bestimmen. Bekannt sind hierfür Standardverfahren wie ein künstliches neuronales Netzwerk, oder bestärkende Lernverfahren wie z.B. genetische Algorithmen. Der Einsatz einer Stützvektormaschine ist aber vorteilhaft, weil anders als bei einem künstlichen neuronalen Netzwerk während des Trainings nicht zu einer Menge von patientenspezifischen Aufnahmewerten die passenden Aufnahmeparameter bekannt sein müssen. Der Einsatz einer Stützvektormaschine ist weiterhin vorteilhaft, weil anders als bei einem bestärkenden Lernverfahren in der Trainingsphase nicht viele unpassende Paare aus patientenspezifischen Aufnahmewerten und Aufnahmeparametern zur Aufnahme eines Bilddatensatzes verwendet werden müssen, insbesondere auch nicht viele zeitaufwändige, aber unbrauchbare Aufnahmen durchgeführt werden müssen.

Ein weiterer Schritt des dargestellten Verfahrens ist die Aufnahme IMG eines Bilddatensatzes 402 mittels eines bildgebenden Geräts 300. Beim bildgebenden Gerät handelt es sich hier um ein medizinisches bildgebendes Gerät 300. Dabei kann es sich in der dargestellten Ausführungsform um einen Computertomographen 300 handeln, aber auch um ein Magnetresonanz-Gerät, um ein Positronen-Emissions-Tomographen, um ein Röntgengerät oder um ein Ultraschallgerät handeln. Die Aufnahme des Bilddatensatzes 402 erfolgt unter der Verwendung des patientenspezifischen Aufnahmewertes und des bestimmten Aufnahmeparameters. Der aufgenommene Bilddatensatz 402 wird dann nach der Aufnahme ausgegeben. Weiterhin werden die Zeitdauer der bildgebenden Aufnahme und die Strahlendosis, welcher der Patient durch die bildgebende Aufnahme ausgesetzt war, mittels einer Ergebnisanzeigeeinheit 403 ausgegeben.

Ein weiterer Schritt des dargestellten Verfahrens ist die Bestimmung einer Qualitätsbewertung QC durch die Bedienperson. Dabei werden die Qualität des aufgenommenen Bilddatensatzes 402, die Zeitdauer der bildgebenden Aufnahme und die Strahlendoses, welcher der Patient durch die bildgebende Aufnahme ausgesetzt war, ausgegeben. Alternativ können auch andere Ergebnisse der Aufnahme für die Bewertung hinzugezogen werden. Im dargestellten Verfahren erfolgt die Bewertung mittels eines binären Parameters, der repräsentiert, ob die Bedienperson mit der Aufnahme zufrieden oder unzufrieden ist. Die Bewertung kann mittels zweier Hardware- oder Softwaretasten 404, 405 erfolgen. Alternativ können auch komplizierte Qualitätsbewertungen erfolgen, beispielsweise durch Schulnoten oder durch einen Schieberegler. Alternativ kann das System die Bewertung der Bedienperson auch davon ableiten, ob eine neue Aufnahme mit gleichem patientenspezifischen Aufnahmewert, aber verändertem Aufnahmeparameter durchgeführt wird. Dieser Fall entspräche einer negativen Qualitätsbewertung, während der Fall, dass keine zusätzliche Aufnahme durchgeführt wird, einer positiven Qualitätsbewertung entspricht.

Ein weiterer Schritt des dargestellten Verfahrens ist das Speichern SAV des Ergebnis-Datensatzes, umfassend den patientenspezifischen Aufnahmewerten, den Aufnahmeparameters und die Qualitätsbewertung. In der dargestellten Ausführungsform erfolgt das Speichern als Ergänzung der Trainings-Datensätze 207.1, 207.2, 207.3 in der Datenbank 206. Das Speichern kann aber auch in der Speichereinheit 203 der Parameterbestimmungseinheit 200 erfolgen.

Ein weiterer Schritt des dargestellten Verfahrens ist das Trainieren TRN der trainierten Abbildungsvorschrift basierend auf den Trainings-Datensätzen 207.1, 207.2, 207.3 und dem Ergebnis-Datensatz. Im dargestellten Verfahren wird hierzu mittels der Stützvektormaschine wenigstens eine erste Hyperfläche bestimmt, welche die zwei Kategorien von Datensätzen trennt. Die trainierte Abbildungsvorschrift kann dann so bestimmt werden, dass zu einem gegebenen patientenspezifischen Aufnahmewert der Aufnahmeparameter derart bestimmt wird, dass er Datenpunkt 512, 602 bestehend aus dem patientenspezifischen Aufnahmewert und dem Aufnahmeparameter einen extremalen Abstand zur Hyperfläche hat. Das Trainieren TRN der trainierten Abbildungsvorschrift erfolgt im dargestellten Verfahren auf einem geographisch entfernten Server. Alternativ kann die trainierte Abbildungsvorschrift aber auch mittels der Parameterbestimmungseinheit 200 trainiert werden. Das Trainieren TRN einer bereits trainierten Abbildungsvorschrift kann auch als kontinuierliches Trainieren bezeichnet werden.

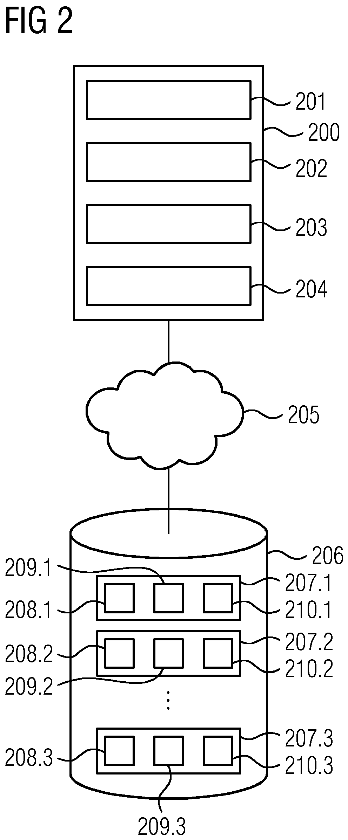

Fig. 2 zeigt eine Parameterbestimmungseinheit 200. In dieser Ausführungsform umfasst die Parameterbestimmungseinheit 200 eine Schnittstelle 201, eine Recheneinheit 202, eine Speichereinheit 203 und eine Ein- und Ausgabeeinheit 204. Bei einer Parameterbestimmungseinheit 200 kann es sich insbesondere um einen Computer, einen Mikrocontroller oder um einen integrierten Schaltkreis handeln. Bei einer Schnittstelle 201 kann es sich um eine Hardware- oder Softwareschnittstelle handeln (beispielsweise PCI-Bus, USB oder Firewire). Eine Recheneinheit 202 kann Hardware-Elemente oder Software-Elemente aufweisen, beispielsweise einen Mikroprozessor oder ein sogenanntes FPGA (engl. Akronym für "Field Programmable Gate Array"). Eine Speichereinheit 203 kann als nicht-dauerhafter Arbeitsspeicher (Random Access Memory, kurz RAM) oder als dauerhafter Massenspeicher (Festplatte, USB-Stick, SD-Karte, Solid State Disc) realisiert sein. Eine Ein- und Ausgabeeinheit 204 kann eine separate Eingabeeinheit (Tastatur, Maus) und eine separate Ausgabeeinheit (Bildschirm, Drucker) umfassen. Sie kann aber auch eine Kombination einer Eingabeeinheit und einer Ausgabeeinheit umfassen, z.B. einen Touchscreen. Die Parameterbestimmungseinheit 200 ist über ein Netzwerk 205 mit einer Datenbank 206 verbunden. Bei dem Netzwerk 205 kann es sich vorzugsweise um das Internet handeln, alternativ aber auch um ein Intranet oder um eine Direktverbindung. Die Datenbank 206 ist vorzugsweise durch einen Server realisiert, der insbesondere auch örtlich getrennt von der Parameterbestimmungseinheit sein kann. Die Datenbank 206 umfasst eine Vielzahl von Datensätzen 207.1, 207.2, 207.3. Jeder der Datensätze 207.1, 207.2, 207.3 umfasst einen patientenspezifischen Aufnahmewert 208.1, 208.2, 208.3, einen Aufnahmeparameter 209.1, 209.2, 209.3 sowie eine Qualitätsbewertung 210.1, 210.2, 210.3.

In der hier gezeigten Ausführungsform ist auf dem Speicher 203 der Parameterbestimmungseinheit 200 wenigstens ein Computerprogramm gespeichert, welches alle Verfahrensschritte des erfindungsgemäßen Verfahrens durchführt, wenn das Computerprogramm auf der Recheneinheit 202 ausgeführt wird. Das Computerprogramm zur Ausführung der Verfahrensschritte des erfindungsgemäßen Verfahrens umfasst Programmcode. Weiterhin kann das Computerprogramm als ausführbare Datei ausgebildet sein und/oder auf einem anderen Rechensystem als der Parameterbestimmungseinheit 200 gespeichert sein. Beispielsweise kann die Parameterbestimmungseinheit 200 so ausgelegt sein, dass das Computerprogramm zum Ausführen des erfindungsgemäßen Verfahrens über ein Intranet oder über das Internet in den Speicher 203 der Parameterbestimmungseinheit 200 geladen wird. Weiterhin ist im gezeigten Ausführungsbeispiel die trainierte Abbildungsvorschrift im Speicher 203 der Parameterbestimmungseinheit gespeichert.

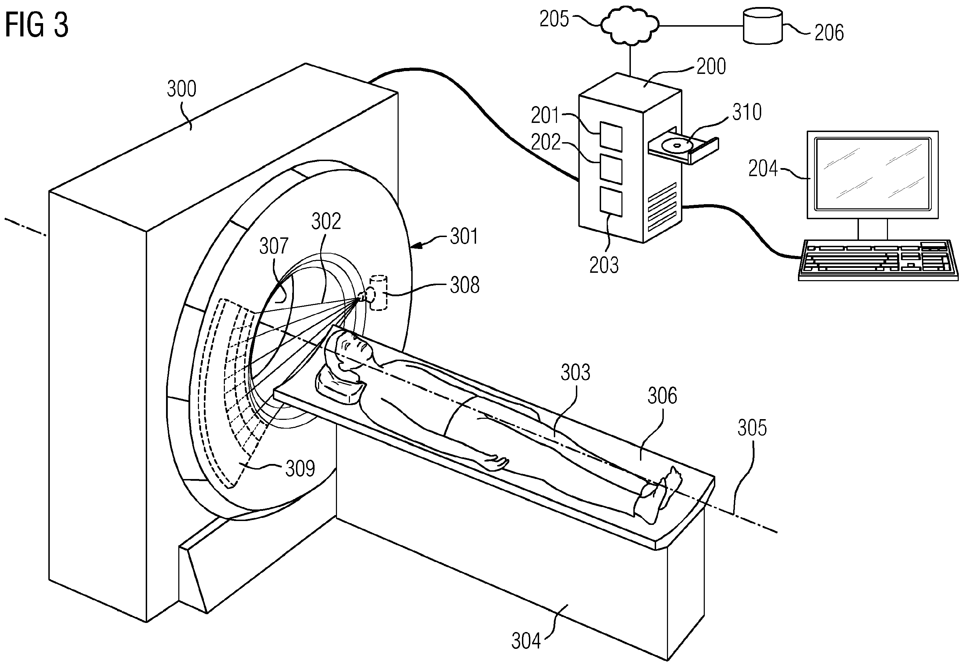

Fig. 3 zeigt bildgebendes Gerät, in der hier gezeigten Ausführungsform ist das bildgebende Gerät ein Computertomographie-Gerät 300. Das hier gezeigte Computertomographie-Gerät 300 verfügt über eine Aufnahmeeinheit 301, umfassend eine Röntgenquelle 308 sowie einen Röntgendetektor 309. Die Aufnahmeeinheit 301 rotiert während der Erfassung von Messdaten um eine Systemachse 305, und die Röntgenquelle 308 emittiert während der Aufnahme Röntgenstrahlen 302. Bei der Röntgenquelle 308 handelt es sich in dem hier gezeigten Beispiel um eine Röntgenröhre. Bei dem Röntgendetektor 309 handelt es sich in dem hier gezeigten Beispiel um einen Zeilendetektor mit mehreren Zeilen.

In dem hier gezeigten Ausführungsbeispiel liegt ein Patient 303 bei der Erfassung von Messdaten auf einer Patientenliege 306. Die Patientenliege 306 ist so mit einem Liegensockel 304 verbunden, dass er die Patientenliege 306 mit dem Patienten 303 trägt. Die Patientenliege 306 ist dazu ausgelegt den Patienten 303 entlang einer Aufnahmerichtung durch die Öffnung 307 der Aufnahmeeinheit 301 zu bewegen. Die Aufnahmerichtung ist in der Regel durch die Systemachse 305 gegeben, um welche die Aufnahmeeinheit 301 bei der Aufnahme von Messdaten rotiert. Bei einer Spiral-Aufnahme wird die Patientenliege 306 kontinuierlich durch die Öffnung 307 bewegt, während die Aufnahmeeinheit 301 um den Patienten 303 rotiert und Messdaten erfasst. Damit beschreiben die Röntgenstrahlen 302 auf der Oberfläche des Patienten 303 eine Spirale. Zur Bestimmung eines Aufnahmeparameters basierend auf einem patientenspezifischen Aufnahmewert verfügt das hier gezeigte Computertomographie-Gerät 300 weiterhin über eine Parameterbestimmungseinheit 200.

In dem hier gezeigten Ausführungsbeispiel ist die Parameterbestimmungseinheit 200 als Computer 200 ausgebildet, weiterhin ist die Schnittstelle 201 als Teil des Computers 200 ausgebildet. Der Computer 200 ist mit einer Ein- und Ausgabeeinheit 204 in Form eines Bildschirms kombiniert mit einer Eingabeeinheit verbunden. Auf dem Bildschirm können die Bilddatensätze 402 in verschiedener Form dargestellt werden, beispielsweise als gerenderte Volumenbilder oder als Schnittbilder. Bei dem Eingabeteil der Ein- und Ausgabeeinheit 204 handelt es sich beispielsweise um eine Tastatur, eine Maus, einen sogenannten "Touch-Screen" oder auch um ein Mikrofon zur Spracheingabe. Mittels der Eingabeeinheit kann ein erfindungsgemäßes Computerprogramm gestartet werden. Die einzelnen Schritte des erfindungsgemäßen Verfahrens können durch die Eingabeeinheit unterstützt werden, beispielsweise kann mittels der Tastatur ein patientenspezifischer Aufnahmewert eingegeben werden, insbesondere die Größe, das Alter, das Gewicht und das Geschlecht des Patienten 303.

Der Computer 200 und die ihm zugeordneten Einheiten können mit einem computerlesbaren Medium 310 zusammenwirken, insbesondere um durch ein Computerprogramm mit Programmcode ein erfindungsgemäßes Verfahren durchzuführen. Weiterhin kann der Programmcode des Computerprogramms auf dem maschinenlesbaren Medium 310 abrufbar gespeichert sein. Insbesondere kann es sich bei dem maschinenlesbaren Medium um eine CD, DVD, Blu-Ray Disc, einen Memory-Stick oder eine Festplatte handeln. Das Computerprogrammprodukt 310 umfasst dabei das Computerprogramm und den entsprechenden Programmcode.

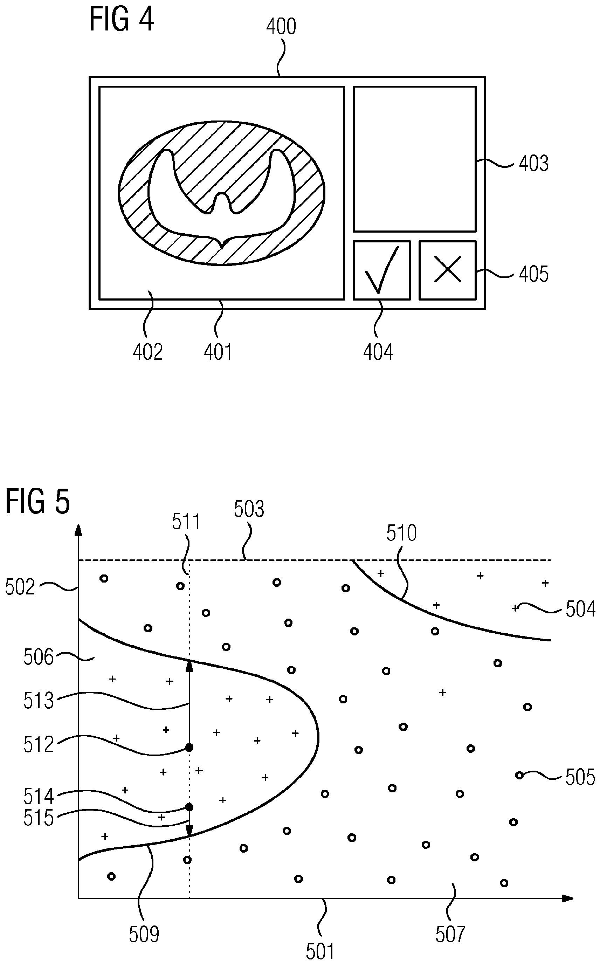

Fig. 4 zeigt ein weiteres Ausführungsbeispiel 400 einer Ein- und Ausgabeeinheit 204. Bei der gezeigten Ein- und Ausgabeeinheit 400 handelt es sich um die Kombination einer Bildanzeigeeinheit 401, auf der ein Bilddatensatz 402 dargestellt werden kann, mit einer Ergebnisanzeigeeinheit 403 und zwei Schaltern 404 und 405. Bei der gezeigten Ein- und Ausgabeeinheit kann es sich insbesondere um einen Touch-Screen handeln, bei der die Bildanzeigeeinheit 401, die Ergebnisanzeigeeinheit 403 und die Schalter 404 und 405 in einem gemeinsamen Bildschirm angezeigt werden. Die Bildanzeigeeinheit 401 kann noch weitere Schaltflächen umfassen, die zur Drehung, Verschiebung, Vergrößerung und/oder Verkleinerung des Bilddatensatzes 402 verwendet werden können. Weiterhin kann die Bildanzeigeeinheit 401 Schaltflächen umfassen, die zur Auswahl eines bestimmten Bildes aus dem Bilddatensatz 402 ausgelegt sind. In der Ergebnisanzeige 403 können insbesondere weitere Daten zur Aufnahme eingeblendet werden, insbesondere die Zeitdauer der Aufnahme des Bilddatensatzes 402 und die Strahlendosis entsprechend der Aufnahme durch einen Patienten 303. Die Ergebnisanzeigeeinheit 403 kann auch in die Bildanzeigeeinheit 401 eingebettet sein. Beim Schalter 404 handelt es sich um eine Schaltfläche, durch deren Drücken eine Bedienperson ihre Zufriedenheit mit der Qualität der Aufnahme des Bilddatensatzes 402 eingeben kann. Beim Schalter 405 handelt es sich um eine Schaltfläche, durch deren Drücken eine Bedienperson ihre Unzufriedenheit mit der Qualität der Aufnahme des Bilddatensatzes 402 eingeben kann. Die Schaltflächen 404 und 405 können auch in die Bildanzeigeeinheit 401 oder die Ergebnisanzeigeeinheit 403 eingebettet sein.

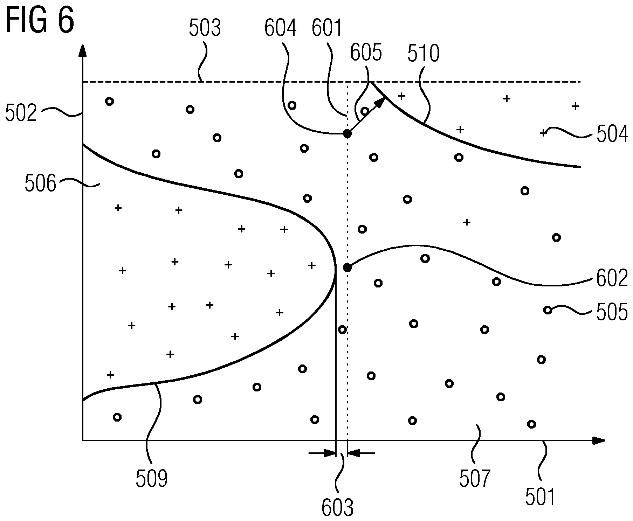

Fig. 5 und Fig. 6 zeigen einen möglichen Parameterraum mit Qualitätsbewertung, sowie ein Ausführungsbeispiel einer trainierten Abbildungsvorschrift. Aufgetragen sind die Koordinatenachse 501 des patientenspezifischen Aufnahmewerts und die Koordinatenachse 502 des Aufnahmeparameters. Im dargestellten Beispiels sind sowohl der patientenspezifische Aufnahmewert als auch der Aufnahmeparameter eindimensionale Größen, der Parameterraum ist also zweidimensional. Weiterhin enthält der Parameterraum Grenzen 503 der einstellbaren Aufnahmeparameter. Die Grenzen 503 sind durch die Eigenschaften des bildgebenden Aufnahmegerätes vorgegeben. Beispielsweise gibt es in einem CT-Gerät einen oberen Grenzwert für die einstellbare Röntgenspannung und/oder den einstellbaren Röntgenstrom. Weiterhin umfasst der Parameterraum Datenpunkte 504, 505, die jeweils einem patientenspezifischen Aufnahmewert und einem Aufnahmeparameter zugeordnet sind. Hierbei wird zwischen Datenpunkten 504 mit positiver Qualitätsbewertung durch die Bedienperson und zwischen Datenpunkten 505 mit negativer Qualitätsbewertung durch die Bedienperson differenziert. Weiterhin umfasst der Parameterraum Unterräume 506, 507, die jeweils nur überwiegend Datenpunkte mit einer einheitlichen Qualitätsbewertung umfassen. Beispielsweise umfasst der Unterraum 506 überwiegend Datenpunkte mit einer positiven Qualitätsbewertung, der Unterraum 507 umfasst überwiegend Datenpunkte 505 mit einer negativen Bewertung. Die Unterräume 506 und 507 sind durch eine Hyperfläche 509 getrennt, weiterhin umfasst der Parameterraum noch weitere Hyperflächen 510, welche weitere Unterräume trennen.

Bei der Bestimmung eines Aufnahmeparameters basierend auf einem patientenspezifischen Aufnahmewert mittels einer trainierten Abbildungsvorschrift wird eine Hyperebene 511, 601 ausgezeichnet, welche alle Datenpunkte mit diesem patientenspezifischen Aufnahmewert enthält. Ein Datenpunkt 512, 514, 602, 604 mit dem zu bestimmenden Aufnahmeparameter und dem vorgegebenen patientenspezifischen Aufnahmewert muss also auf der Hypereben 511, 601 liegen.

In einem ersten Fall, dargestellt in Fig. 5, hat die Hyperebene 511 gemeinsame Datenpunkte mit einem Unterraum 506, der überwiegend Datenpunkte 504 mit positiver Qualitätsbewertung enthält. Ein Datenpunkt 512, der zum mittels der trainierten Abbildungsvorschrift bestimmten Aufnahmeparameter 502 gehört, liegt dann in einem Unterraum 506 mit Datenpunkten mit überwiegend positiver Qualitätsbewertung, und hat maximalen Abstand 513 zu der Hyperfläche 509, die den Unterraum 506 begrenzt, und zu allen anderen Hyperflächen. Ein anderer, nicht geeigneter Datenpunkt 514 hat einen nicht-maximalen Abstand 515 zu der Hyperfläche 509.

In einem zweiten Fall, dargestellt in Fig. 6, hat die Hyperebene 601 keine gemeinsamen Datenpunkte mit einem Unterraum 506, der überwiegend Datenpunkte 504 mit positiver Qualitätsbewertung enthält, sondern liegt ausschließlich in einem Unterraum 507, der überwiegend Datenpunkt 505 mit negativer Qualitätsbewertung enthält. Ein Datenpunkt 602, der zum mittels der trainierten Abbildungsvorschrift bestimmten Aufnahmeparameter 502 gehört, liegt dann im Unterraum 507 mit Datenpunkten mit überwiegend negativer Qualitätsbewertung, und hat minimalen Abstand 603 zu der nächstliegenden Hyperfläche 509, die den Unterraum 507 begrenzt. Ein anderer, nicht geeigneter Datenpunkt 604 hat einen nicht-maximalen Abstand 605 zu der nächstliegenden Hyperfläche 510.

**Patentansprüche**

1. Verfahren zum Bestimmen eines Aufnahmeparameters, umfassend folgende Verfahrensschritte, durchgeführt mittels einer Parameterbestimmungseinheit:

   - Empfangen (REC) eines patientenspezifischen Aufnahmewertes für eine Aufnahme eines Bilddatensatzes (402),
   - Bestimmen (DET) eines Aufnahmeparameters durch Anwendung einer trainierten Abbildungsvorschrift auf den patientenspezifischen Aufnahmewert,

   wobei die trainierte Abbildungsvorschrift auf einer Mehrzahl von Trainings-Datensätzen (207.1, 207.2, 207.3) basiert, wobei die Trainings-Datensätze (207.1, 207.2, 207.3) jeweils wenigstens einen patientenspezifische Trainings-Aufnahmewert (208.1, 208.2, 208.3) und wenigstens einen Trainings-Aufnahmeparameter (209.1, 209.2, 209.3) sowie wenigstens eine Trainings-Qualitätsbewertung (210.1, 210.2, 210.3) umfassen.

2. Verfahren nach Anspruch 1, weiterhin umfassend folgende Verfahrensschritte:

   - Aufnehmen (IMG) des Bilddatensatzes (402) unter Verwendung des Aufnahmeparameters mittels eines bildgebenden Geräts (300),
   - Bestimmen (QC) eines Ergebnis-Datensatzes, umfassend eine Qualitätsbewertung des Bilddatensatzes (402) und den patientenspezifischen Aufnahmewert sowie den Aufnahmeparameters,

   - Speichern (SAV) des Ergebnis-Datensatzes.

3. Verfahren nach Anspruch 2, weiterhin umfassend folgenden Verfahrensschritt:

   - Trainieren (TRN) der trainierten Abbildungsvorschrift basierend auf den Trainings-Datensätzen (207.1, 207.2, 207.3) sowie dem Ergebnis-Datensatz.

4. Verfahren nach Anspruch 2 oder 3, wobei die Qualitätsbewertung ein Maß für wenigstens eines der folgenden Merkmale ist:

   - Qualität des Bilddatensatzes (402),
   - Zeitdauer der Aufnahme des Bilddatensatzes (402),
   - Strahlendosis entsprechend der Aufnahme des Bilddatensatzes (402) vom Patienten (303).

5. Verfahren nach einem der Ansprüche 1 bis 4, wobei die Trainings-Qualitätsbewertung (210.1, 210.2, 210.3) ein Maß für wenigstens eines der folgenden Merkmale eines Trainings-Bilddatensatzes ist:

   - Qualität des Trainings-Bilddatensatzes,
   - Zeitdauer der Aufnahme des Trainings-Bilddatensatzes,
   - Strahlendosis entsprechend der Aufnahme des Trainings-Bilddatensatzes von einem Patienten (303).

6. Verfahren nach einem der Ansprüche 1 bis 5, wobei die Qualitätsbewertung und/oder die Trainings-Qualitätsbewertung (210.1, 210.2, 210.3) einen diskreten Parameter umfassen, der nur endlich viele Werte annehmen kann.

7. Verfahren nach Anspruch 6, wobei der diskrete Parameter genau zwei verschiedene Werte annehmen kann.

8. Verfahren nach Anspruch 6 oder 7, wobei die trainierte Abbildungsvorschrift mittels einer Stützvektormaschine derart auf den Trainings-Datensätzen (207.1, 207.2, 207.3) basiert, dass wenigstens ein Teil der Trainings-Datensätze (207.1, 207.2, 207.3) mittels des diskreten Parameters der Trainings-Qualitätsbewertung in wenigstens eine erste Kategorie und eine zweite Kategorie sortiert werden, dass im Parameterraum aller Kombinationen patientenspezifischer Aufnahmewerte und Aufnahmeparameter wenigstens eine erste Hyperfläche (509) bestimmt wird, welche die erste und die zweite Kategorie trennt, und dass die trainierte Abbildungsvorschrift wenigstens mittels der ersten Hyperfläche (509) bestimmt wird.

**9.** Verfahren nach Anspruch 8, wobei die Abbildungsvorschrift derart durch die erste Hyperfläche (509) bestimmt wird, dass sie einen patientenspezifischen Aufnahmewert derart auf den Aufnahmeparameter abbildet, dass der Abstand (513, 603) zwischen der ersten Hyperfläche (509) und einem Datenpunkt (512, 602) extremal wird, wobei der Datenpunkt (512, 602) aus dem patientenspezifischen Aufnahmewert und dem bestimmten Aufnahmeparameter besteht.

**10.** Parameterbestimmungseinheit (200) zum Bestimmen eines Aufnahmeparameters, umfassend folgende Einheiten:

   - Schnittstelle (201), ausgebildet zum Empfangen (REC) eines patientenspezifischen Aufnahmewertes für eine Aufnahme eines Bilddatensatzes (402),
   - Recheneinheit (202), ausgebildet zum Bestimmen (DET) eines Aufnahmeparameters durch Anwendung einer trainierten Abbildungsvorschrift auf den patientenspezifischen Aufnahmewert, wobei die trainierte Abbildungsvorschrift auf einer Mehrzahl von Trainings-Datensätzen (207.1, 207.2, 207.3) basiert, wobei die Trainings-Datensätze (207.1, 207.2, 207.3) jeweils wenigstens einen patientenspezifische Trainings-Aufnahmewert (208.1, 208.2, 208.3) und wenigstens einen Trainings-Aufnahmeparameter (209.1, 209.2, 209.3) sowie wenigstens eine Trainings-Qualitätsbewertung (210.1, 210.2, 210.3) umfassen.

**11.** Parameterbestimmungseinheit (200) nach Anspruch 10, weiterhin umfassend eine Speichereinheit (203) sowie eine Aus- und/oder Eingabeeinheit (204), weiterhin ausgebildet das Verfahren nach einem der Ansprüchen 2 bis 9 durchzuführen.

**12.** Computerprogrammprodukt mit einem Computerprogramm, welches direkt in den Speicher (203) einer Parameterbestimmungseinheit (200) ladbar ist, mit Programmabschnitten, um alle Schritte des Verfahrens nach einem der Ansprüche 1 bis 9 auszuführen, wenn die Programmabschnitte von der Parameterbestimmungseinheit (200) ausgeführt werden.

**13.** Computerlesbares Medium (310), auf welchem von einer Parameterbestimmungseinheit (200) lesbare und ausführbare Programmabschnitte gespeichert sind, um alle Schritte des Verfahrens nach einem der Ansprüche 1 bis 9 auszuführen, wenn die Programmabschnitte von der Parameterbestimmungseinheit (200) ausgeführt werden.

**14.** Bildgebendes Gerät (300), umfassend eine Parameterbestimmungseinheit (200) nach Anspruch 10 oder 11.

FIG 1

```
┌──────────────┐
│              │ ╌ REC
└──────┬───────┘
       ↓
┌──────────────┐
│              │ ╌ DET
└──────┬───────┘
       ↓
┌──────────────┐
│              │ ╌ IMG
└──────┬───────┘
       ↓
┌──────────────┐
│              │ ╌ QC
└──────┬───────┘
       ↓
┌──────────────┐
│              │ ╌ SAV
└──────┬───────┘
       ↓
┌──────────────┐
│              │ ╌ TRN
└──────────────┘
```

# FIG 2

201
200
202
203
204

205

206
209.1
207.1
208.1
210.1
208.2
207.2
210.2
209.2
208.3
207.3
210.3
209.3

FIG 3

EP 3 309 792 A1

FIG 4

400

403

405

402    401    404

FIG 5

511    503    510

502    504

506

513

512    505

514
515

509    507

501

FIG 6

**Europäisches Patentamt**
**European Patent Office**
**Office européen des brevets**

## EUROPÄISCHER RECHERCHENBERICHT

Nummer der Anmeldung

EP 17 19 0742

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DE 10 2011 002928 A1 (SIEMENS AG [DE]) 26. Juli 2012 (2012-07-26) * das ganze Dokument * * insbesondere * * Par 0015-0023, 0036; Ansprüche 1-6; Abbildung 1 * ----- | 1-14 | INV. G16H30/20 G16H40/63 |
| X | DE 10 2014 102080 A1 (ZEISS CARL AG [DE]) 20. August 2015 (2015-08-20) * das ganze Dokument * * insbesondere * * 0007-0016, 0063, 0075, 0096; Ansprüche 1-4,7; Abbildungen 1-5 * ----- | 1-14 | |

RECHERCHIERTE SACHGEBIETE (IPC)

G06F
G16H

Der vorliegende Recherchenbericht wurde für alle Patentansprüche erstellt

| Recherchenort | Abschlußdatum der Recherche | Prüfer |
|---|---|---|
| München | 1. März 2018 | Rákossy, Zoltán |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

&: Mitglied der gleichen Patentfamilie, übereinstimmendes Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**

EP 17 19 0742

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

01-03-2018

| Im Recherchenbericht angeführtes Patentdokument | Datum der Veröffentlichung | Mitglied(er) der Patentfamilie | Datum der Veröffentlichung |
|---|---|---|---|
| DE 102011002928 A1 | 26-07-2012 | DE 102011002928 A1<br>US 2012190962 A1 | 26-07-2012<br>26-07-2012 |
| DE 102014102080 A1 | 20-08-2015 | KEINE | |

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

• **INGO STEINWART ; ANDREAS CHRISTMANN.** Support Vector Machines. Springer, 2008